(19) Europäisches Patentamt
European Patent Office
Office européen des brevets

(11) EP 1 092 707 B1

(12) FASCICULE DE BREVET EUROPEEN

(45) Date de publication et mention de la délivrance du brevet:
**02.07.2008 Bulletin 2008/27**

(51) Int Cl.:
***C07C 247/04*** *(2006.01)* ***C06B 45/06*** *(2006.01)*

(21) Numéro de dépôt: **00402593.8**

(22) Date de dépôt: **20.09.2000**

(54) **Polyazotures de glycidyle comportant une terminaison acyloxy et une terminaison azide**

Polyglycidylazid Verbindungen mit einer endständigen Acyloxy Gruppe und einer endständigen Azido Gruppe

Polyglycidylazide having a terminal acyloxy group and a terminal azido group

(84) Etats contractants désignés:
**AT BE CH CY DE DK ES FI FR GB GR IE IT LI LU MC NL PT SE**

(30) Priorité: **15.10.1999 FR 9912865**

(43) Date de publication de la demande:
**18.04.2001 Bulletin 2001/16**

(73) Titulaire: **SNPE Matériaux Energétiques**
**75004 Paris (FR)**

(72) Inventeurs:
- **Bouchez, Jean-Marc**
**91610 Ballancourt sur Essonne (FR)**
- **Graindorge, Hervé**
**91710 Vert-Le-Petit (FR)**
- **Soriaux, Claude**
**91540 Mennecy (FR)**

(74) Mandataire: **Le Roux, Martine et al**
**Cabinet Beau de Loménie**
**158, rue de l'Université**
**75340 Paris Cédex 07 (FR)**

(56) Documents cités:
**FR-A- 2 707 979**
**US-A- 4 970 326**
**US-A- 5 565 650**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).



Printed by Jouve, 75001 PARIS (FR)

## Description

La présente invention se situe dans le domaine des compositions pyrotechniques solides, notamment dans celui des propergols solides, des explosifs composites et des poudres propulsives pour armes.

Elle a plus précisément pour objet de nouveaux plastifiants énergétiques utilisables dans les compositions pyrotechniques solides précitées.

Ces compositions pyrotechniques sont généralement obtenues par coulée dans un moule puis réticulation d'une pâte essentiellement constituée d'un liant réticulable, d'un système réticulant de ce liant, et de charges oxydantes et/ou réductrice.

Des plastifiants sont souvent incorporés pour améliorer la mise en oeuvre et les propriétés mécaniques du produit fini, notamment aux basses températures. De préférence, ces plastifiants sont des composés énergétiques miscibles et non réactifs avec les autres constituants de la pâte, notamment avec le liant et le réticulant.

Il est connu d'utiliser dans ce but des nitrates d'alcools tels que la nitroglycérine et le dinitrate d'éthylène glycol, mais ces composés sont particulièrement sensibles, détonables et dangereux à manipuler.

Le brevet FR 2 707 979 décrit par ailleurs des polyazotures de glycidyle (PAG) terminés par des fonctions azides utilisables comme plastifiants énergétiques dans de telles compositions pyrotechniques, mais il s'avère que ces PAG sont également détonables et qu'ils doivent être classés en classe 1-1 selon l'article 4 de l'arrêté interministériel du 26 septembre 1980 relatif au classement des substances explosibles (publication au Journal Officiel de la République Française le 2 octobre 1980) et la circulaire interministérielle d'application de cet arrêté en date du 8 mai 1981, c'est à dire dans une classe correspondant à des produits particulièrement sensibles tels que l'hexogène, l'octogène et la nitroglycérine, avec toutes les contraintes que cela entraîne concernant la sécurité du transport, du stockage et de la mise en oeuvre, donc sur les coûts qui sont en conséquence élevés.

Le brevet US 4 970 326 décrit des PAG diacétates utilisables comme plastifiants dans les compositions pyrotechniques solides, mais ces composés, certes non détonables, sont nettement moins énergétiques (teneur en azote de l'ordre de 32-33%) que les PAG à terminaisons azides.

L'homme du métier, qui a le souci permanent d'améliorer la sécurité des procédés et de diminuer les coûts de fabrication, recherche donc de nouveaux plastifiants énergétiques comportant moins de risques pyrotechniques que les PAG à terminaisons azides de l'état de la technique, notamment des plastifiants qui seraient à la fois énergétiques et non détonables.

La présente invention a pour objet de tels plastifiants, plus précisément de nouveaux polyazotures de glycidyle énergétiques (teneur en azote voisine de 41%), liquides dans les conditions normales de température et de pression, utilisables comme plastifiants énergétiques dans les compositions pyrotechniques solides, et non détonables, c'est à dire qu'ils sont notamment classés en classe 1-3 selon l'arrêté interministériel précité, c'est à dire dans une classe correspondant à des produits à risques atténués tels que les propergols et les poudres pour armes à feu, et non pas en classe 1-1 comme les PAG détonables décrits dans FR 2 707 979.

Les nouveaux polyazotures de glycidyle selon l'invention répondent à la formule générale (I)

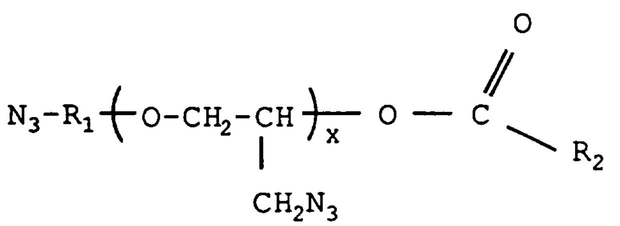

(I)

dans laquelle :

- $R_1$ représente une chaine alkylène comportant 2 à 4 atomes de carbone, de préférence $-(CH_2)_2-$,
- $R_2$ représente une chaine alkyle comportant 1 à 4 atomes de carbone, de préférence $-CH_3$,
- x représente un nombre entier tel que $4 \leq x \leq 10$, de préférence tel que $6 \leq x \leq 8$.

Ces nouveaux PAG de formule (I) selon l'invention peuvent être obtenus en 3 étapes à partir d'épichlorhydrine.

Dans une première étape, on synthétise une polyépichlorhydrine à terminaison hydroxyle de formule générale (II)

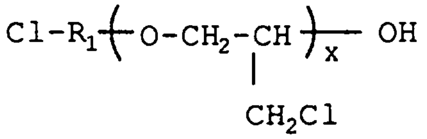

(II)

dans laquelle x et $R_1$ ont la signification précitée, par polymérisation, généralement en milieu solvant organique, de préférence halogéné, d'épichlorhydrine en présence d'un alcool amorceur de formule générale (III) $Cl\text{-}R_1\text{-}OH$ dans laquelle $R_1$ a la signification précitée.

Une telle réaction est bien connue de l'homme du métier et est généralement conduite en présence d'un catalyseur de type acide de Lewis, par exemple un éthérate de $BF_3$.

Dans une seconde étape, on synthétise une polyépichlorhydrine à terminaison acyloxy de formule générale (IV)

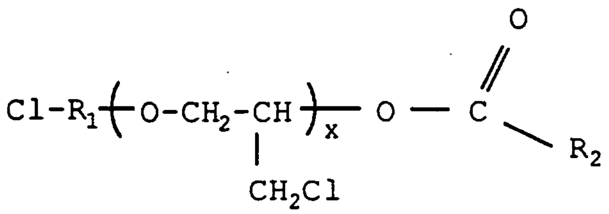

(IV)

dans laquelle $R_1$, $R_2$ et x ont la signification précitée, par réaction, en présence d'un catalyseur organique basique, de la polyépichlorhydrine à terminaison hydroxyle précitée de formule (II) avec un agent acylant de formule générale (V)

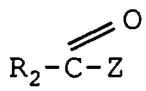

dans laquelle $R_2$ a la signification précitée et Z représente un groupe hydroxyle, halogénure (Cl ou Br de préférence), ou acyloxy.

De façon particulière préférée, l'agent acylant de formule (V) est un anhydride de formule

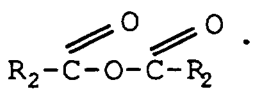

L'agent acylant est en général utilisé en très large excès molaire par rapport aux fonctions hydroxyles. On peut par exemple utiliser un rapport molaire compris entre 2 et 8.

Le catalyseur organique basique est de préférence choisi dans le groupe constitué par les pyridines et les imidazoles. La pyridine et la 1-méthylimidazole sont particulièrement préférées.

Comme exemples d'autres catalyseurs convenables, on peut citer les trialkylamines, notamment la triéthylamine.

La quantité généralement utilisée de catalyseur est comprise entre 10% et 50% molaires par rapport aux fonctions hydroxyles à estérifier.

Selon une variante préférée, cette seconde étape est effectuée en milieu solvant organique, de préférence halogéné, plus particulièrement chloré, tel que $CHCl_3$, $CCl_4$, $CH_2Cl_2$ et $CH_2Cl\text{-}CH_2Cl$.

La température de cette réaction d'acylation est de préférence comprise entre 40°C et 100°C. La plage de température 70°C-90°C est particulièrement préférée et on opère de préférence à reflux du solvant.

Après réaction de la polyépichlorhydrine à terminaison hydroxyle de formule (II) avec l'agent acylant de formule (V), la polyépichlorhydrine à terminaison acyloxy de formule générale (IV) formée est isolée du milieu réactionnel, puis identifiée selon les méthodes classiques d'analyses, physiques, chimiques, chromatographiques et spectrométriques.

Par spectrométrie infra-rouge, on constate notamment, comparativement au spectre de la polyépichlorhydrine à terminaison hydroxyle de départ, la disparition des bandes hydroxyles, l'apparition d'une bande carbonyle, alors que la bande C-Cl est toujours présente.

Dans une troisième étape, on effectue une azidation des fonctions chlorures de la polyépichlorhydrine à terminaison acyloxy précitée de formule générale (IV) obtenue à la seconde étape, de préférence au moyen d'un azoture alcalin.

On a constaté, de façon particulièrement inattendue, que la fonction acyle portée par les polyépichlorhydrines intermédiaires de formule générale (IV) était inerte vis à vis des réactifs d'azidation et qu'elle n'était donc pas, même partiellement, transformée en fonction azoture, alors que l'état de la technique, et notamment US 4 970 326 précité, incite l'homme du métier à effectuer la réaction d'azidation avant la réaction d'acylation, avec les inconvénients que cela entraîne au niveau sécurité (nombre plus élevé d'étapes pyrotechniques), donc au niveau des coûts.

Selon l'invention, seule la troisième et dernière étape est une étape pyrotechnique, ce qui limite considérablement les risques pyrotechniques et le coût global.

Selon une variante préférée, cette troisième étape d'azidation est réalisée au moyen d'un azoture alcalin, par exemple $NaN_3$.

Selon une autre variante préférée, cette étape d'azidation est effectuée en milieu solvant organique, de préférence polaire, tel que le diméthylsulfoxyde (DMSO) et la diméthylformamide (DMF).

La température de la réaction d'azidation est de préférence comprise entre 70°C et 110°C, plus particulièrement entre 90°C et 100°C.

Après réaction, puis refroidissement du milieu réactionnel, les nouveaux PAG de formule (I) selon l'invention peuvent être isolés du milieu par exemple par précipitation en ajoutant de l'eau au milieu, puis purifiés par dissolution dans un solvant chloré tel que le chlorure de méthylène, puis séchage, filtration et évaporation du solvant.

Le produit purifié peut ensuite être identifié selon les méthodes classiques d'analyses, physiques, chimiques, chromatographiques et spectrométriques.

Par spectrométrie infra-rouge, comparativement au spectre de la polyépichlorhydrine à terminaison acyloxy de départ, on constate notamment l'apparition des bandes caractéristiques de la liaison $C\text{-}N_3$, la disparition de la bande C-Cl, alors que la bande carbonyle est toujours présente.

La présente invention, outre les nouveaux PAG précités de formule (I), a également pour objet toute composition pyrotechnique solide, notamment tout propergol solide, tout explosif composite et toute poudre propulsive pour armes à feu, comprenant un polyazoture de glycidyle précité de formule (I), ainsi que toute utilisation d'un tel PAG comme plastifiant énergétique dans lesdites compositions pyrotechniques solides.

Les exemples non limitatifs suivants illustrent l'invention et les avantages qu'elle procure.

Exemple 1:

Obtention d'une polyépichlorhydrine à terminaison hydroxyle de formule (II) avec $R_1 = -(CH_2)_2-$.

Dans un réacteur double enveloppe de 500 ml équipé d'une agitation mécanique, d'une sonde de température et d'un réfrigérant à eau, on introduit 380 g de 1,2-dichloroéthane (solvant), 62,85 g (0,78 mol) de 2-chloroéthanol (amorceur) et 5,35 g (3,8 $10^{-2}$ mol) d'éthérate de trifluorure de bore (catalyseur).

On porte le mélange à 30°C, puis on additionne goutte à goutte, en 6 heures, 650 g (7,03 mol) d'épichlorhydrine, tout en maintenant la température à 30°C.

Après réaction, on verse le milieu réactionnel dans 0,75 l d'eau contenant 15 g de $K_2\,CO_3$.

Après agitation durant 20 min environ, puis décantation, on soutire la phase aqueuse, puis on lave une seconde fois la phase organique à l'eau additionnée de $K_2\,CO_3$, puis une troisième fois à l'eau pure.

Après évaporation du solvant, on recueille 685 g (rendement 96%) d'un liquide incolore dont les principales caractéristiques mesurées sont les suivantes :

- Taux d'hydroxyle : 1,11 eqOH/kg
- Analyse par chromatographie par perméation de gel avec étalonnage PPG :

$$\overline{Mn} = 700$$

$$\overline{Mp} = 800$$

$$I = \frac{\overline{Mp}}{\overline{Mn}} = 1,15$$

- Spectre IR en conformité avec la formule (II) avec notamment présence de bandes OH à 3472 $cm^{-1}$ et C-Cl à 747 $cm^{-1}$.

Exemple 2 :

Obtention d'une polyépichlorhydrine à terminaison acétyloxy de formule (IV) avec $R_1$ = $-(CH_2)_2-$ et $R_2$ = $-CH_3$.
Dans un réacteur double enveloppe de 1 l équipé d'une agitation mécanique, d'une sonde de température et d'un réfrigérant à eau, on dissout 200 g de la polyépichlorhydrine à terminaison hydroxyle obtenue selon l'exemple 1 dans 300 ml de 1,2-dichloroéthane, puis on ajoute, à la température ambiante (18°C environ), 120 g (1,18 mol) d'anhydride acétique.
On ajoute ensuite 10 ml (0,125 mol) de 1-méthylimidazole, puis on porte le milieu réactionnel au reflux du 1,2-dichloroéthane (81°C) et maintient cette température pendant 3 h.
Après refroidissement à la température ambiante, on lave le milieu avec 300 ml d'eau, puis on le sèche sur sulfate de magnésium.
Après filtration, puis évaporation du solvant, on récupère 170 g (rendement 81%) d'un liquide incolore dont le Spectre IR présente une bande carbonyle à 1743 $cm^{-1}$, une bande C-Cl à 747 $cm^{-1}$, mais ne présente pas de bande hydroxyle.

Exemple 3 :

Obtention d'un polyazoture de glycidyle à terminaison acétyloxy de formule (I) selon l'invention avec $R_1$ = $-(CH_2)_2$ - et $R_2$ = $-CH_3$.
Dans un réacteur double enveloppe de 1 l équipé d'une agitation mécanique, d'une sonde de température et d'un réfrigérant à eau, on introduit 340 ml de DMSO et 170 g de la polyépichlorhydrine à terminaison acétate obtenue selon l'exemple 2.
On porte la température du milieu vers 75°C, puis on introduit 139,6 g (2,15 mol) d'azoture de sodium, par portions de façon à maintenir la température du milieu réactionnel entre 90 et 95°C, puis on maintient la température du milieu à 95°C durant 24 h.
Après refroidissement à la température ambiante, on ajoute 500 ml d'eau. Un produit précipite que l'on recueille par filtration, puis lave par 3 fois 500 ml d'eau afin d'éliminer le DMSO résiduel, puis dissout dans 500 ml de chlorure de méthylène.
Après séchage sur sulfate de magnésium, puis filtration, puis évaporation du solvant, on recueille 163 g (rendement 90%) d'une huile jaunâtre dont les principales caractéristiques mesurées sont les suivantes :

- Spectre IR en conformité avec la formule (I) avec notamment présence de bandes à 1282 $cm^{-1}$, 2101 $cm^{-1}$, 2520 $cm^{-1}$ et 3362 $cm^{-1}$ caractéristiques de la liaison $C-N_3$, ainsi que d'une bande carbonyle à 1743 $cm^{-1}$. Ce spectre ne présente pas de bande hydroxyle, ni de bande caractéristique de la liaison C-Cl à 747 $cm^{-1}$.
- Analyse par chromatographie par perméation de gel avec étalonnage PPG :

• $\overline{Mn} = 740$

• $\overline{Mp} = 850$

• $I = \frac{\overline{Mp}}{\overline{Mn}} = 1,15$

- Teneur en azote : 41,1%
- Masse volumique à 20°C : 1,269 g/$cm^3$
- Taux de chlore résiduel : < 0,2%
- Taux d'eau résiduelle : 0,05%
- Taux de DMSO résiduel : 0,1%
- Sensibilité au choc à 20°C et 40°C (appareil Julius Peters) : absence de détonation à 50J (30 essais à chaque température)
- Stabilité sous vide (100°C, 193 h) : 2,8 $cm^3$/g
- Tests de classement selon l'arrêté interministériel et la circulaire d'application précités : classement en classe 1-3.

A titre comparatif, ces mêmes tests ont été réalisés sur un échantillon de PAG tel que celui décrit dans FR 2 707 979. Les résultats montrent qu'un tel PAG doit être classé en classe 1-1.

## Revendications

1. Polyazotures de glycidyle de formule générale (I)

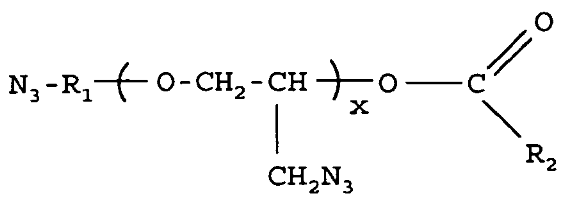

(I)

dans laquelle :

- $R_1$ représente une chaîne alkylène comportant 2 à 4 atomes de carbone,
- $R_2$ représente une chaîne alkyle comportant 1 à 4 atomes de carbone,
- x représente un nombre entier tel que $4 \leq x \leq 10$.

2. Polyazotures de glycidyle selon la revendication 1, **caractérisés en ce que** $R_1$ représente $-(CH_2)_2-$ et **en ce que** $R_2$ représente $-CH_3$.

3. Polyazotures de glycidyle selon la revendication 1 ou 2, **caractérisés en ce que** x est tel que $6 \leq x \leq 8$.

4. Procédé de synthèse de polyazotures de glycidyle de formule (I)

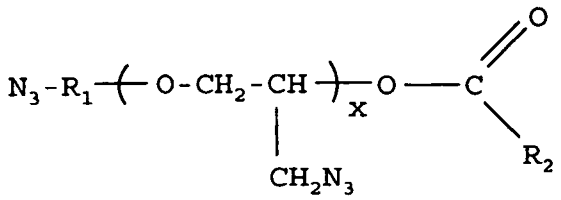

(I)

dans laquelle :

- $R_1$ représente une chaîne alkylène comportant 2 à 4 atomes de carbone,
- $R_2$ représente une chaîne alkyle comportant 1 à 4 atomes de carbone,
- x représente un nombre entier tel que $4 \leq x \leq 10$, **caractérisé en ce que** :
- dans une première étape, on synthétise une polyépichlorhydrine à terminaison hydroxyle de formule générale (II)

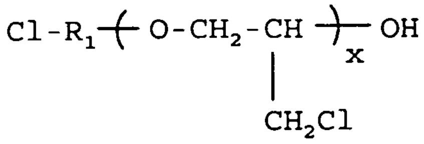

(II)

dans laquelle x et $R_1$ ont la signification précitée, par polymérisation d'épichlorhydrine en présence d'un alcool amorceur de formule générale (III) $Cl-R_1-OH$ dans laquelle $R_1$ a la signification précitée; puis **en ce que**,

- dans une seconde étape, on synthétise une polyépichlorhydrine à terminaison acyloxy de formule générale (IV)

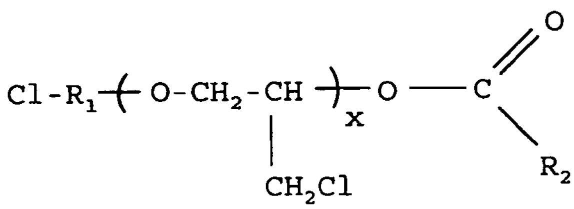

(IV)

dans laquelle $R_1$, $R_2$ et x ont la signification précitée, par réaction, en présence d'un catalyseur organique basique, de la polyépichlorhydrine à terminaison hydroxyle précitée de formule (II) avec un agent acylant de formule générale (V)

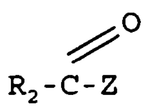

dans laquelle $R_2$ a la signification précitée et Z représente un groupe hydroxyle, halogénure ou acyloxy, puis **en ce que**,

- dans une troisième étape, on effectue une azidation des fonctions chlorures de la polyépichlorhydrine à terminaison acyloxy précitée de formule générale (IV) obtenue à la seconde étape.

5. Procédé de synthèse selon la revendication 4, **caractérisé en ce que** l'agent acylant de formule générale (V) est un anhydride de formule

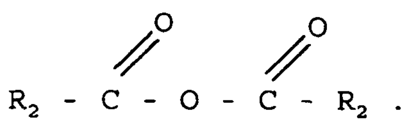

6. Procédé de synthèse selon la revendication 4 ou 5, **caractérisé en ce que** les 3 étapes réactionnelles sont effectuées en milieu solvant organique.

7. Procédé de synthèse selon l'une quelconque des revendications 4 à 6, **caractérisé en ce que** ledit catalyseur organique basique est choisi dans le groupe constitué par les pyridines et les imidazoles.

8. Procédé de synthèse selon l'une quelconque des revendications 4 à 7, **caractérisé en ce que** l'azidation est réalisée au moyen d'un azoture alcalin.

9. Composition pyrotechnique solide, **caractérisée en ce qu'**elle comprend un polyazoture de glycidyle selon l'une quelconque des revendications 1 à 3 ou préparé selon le procédé de synthèse de l'une quelconque des revendications 4 à 8.

**10.** Utilisation d'un polyazoture de glycidyle, selon l'une quelconque des revendications 1 à 3 ou préparé selon l'une quelconque des revendications 4 à 8, comme plastifiant énergétique dans des compositions pyrotechniques solides.

## Claims

**1.** Polyglycidylazides of general formula (I)

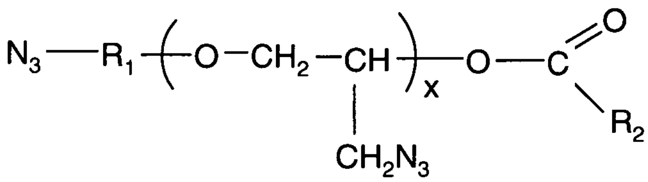

(I)

in which:

- $R_1$ represents an alkylene chain comprising 2 to 4 carbon atoms,
- $R_2$ represents an alkyl chain comprising 1 to 4 carbon atoms,
- x represents an integer such that $4 \leq x \leq 10$.

**2.** Polyglycidylazides according to claim 1, **characterized in that** $R_1$ represents $-(CH_2)_2-$ and $R_2$ represents $-CH_3$.

**3.** Polyglycidylazides according to claim 1 or 2, **characterized in that** x is such that $6 \leq x \leq 8$.

**4.** Process for the synthesis of polyglycidylazides of formula (I)

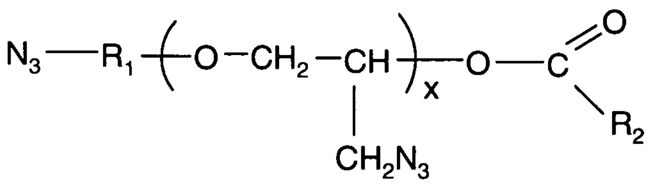

(I)

in which:

- $R_1$ represents an alkylene chain comprising 2 to 4 carbon atoms,
- $R_2$ represents an alkyl chain comprising 1 to 4 carbon atoms,
- x represents an integer such that $4 \leq x \leq 10$,

**characterized in that**:
+ in a first stage, a polyepichlorohydrin with a hydroxyl terminal group of general formula (II)

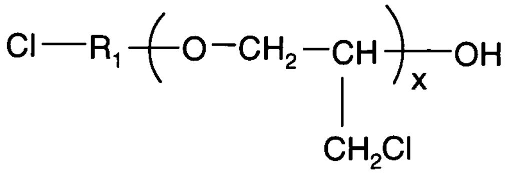

(II)

in which x and $R_1$ have the abovementioned meanings, is synthesized by polymerization of epichlorohydrin in the presence of an initiating alcohol of general formula (III) $Cl-R_1-OH$, in which $R_1$ has the above mentioned meaning;

**in that** then,

+ in a second stage, a polyepichlorohydrin with an acyloxy terminal group of general formula (IV)

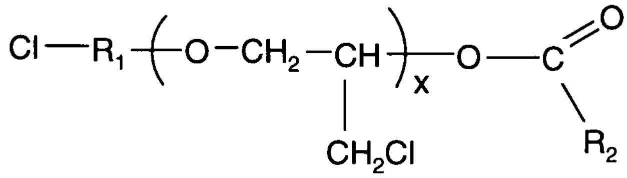

(IV)

in which $R_1$, $R_2$ and x have the above mentioned meanings, is synthesized by reaction, in the presence of a basic organic catalyst, of the above mentioned polyepichlorohydrin with a hydroxyl terminal group of formula (II) with an acylating agent of general formula (V)

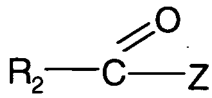

in which $R_2$ has the abovementioned meaning and Z represents a hydroxyl, halide or acyloxy group; and **in that** then,

+ in a third stage, an azidation is carried out on the chloride functional groups of the above mentioned polyepichlorohydrin with an acyloxy terminal group of general formula (IV) obtained in the second stage.

5. The synthesis process according to claim 4, **characterized in that** the acylating agent of general formula (IV) is an anhydride of formula

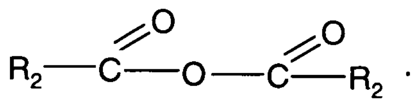

6. The synthesis process according to claim 4 or 5, **characterized in that** the 3 reaction stages are carried out in an organic solvent medium.

7. The synthesis process according to any one of claims 4 to 6, **characterized in that** the basic organic catalyst is chosen from the group consisting of pyridines and imidazoles.

8. The synthesis process according to any one of claims 4 to 7, **characterized in that** the azidation is carried out by means of an alkali metal azide.

9. Solid pyrotechnic composition, **characterized in that** it comprises a polyglycidylazide according to any one of claims 1 to 3 or prepared according to the synthesis process according to any one of claims 4 to 8.

10. Use of a polyglycidylazide according to any one of claims 1 to 3 or prepared according to the synthesis process according to any one of claims 4 to 8 as energetic plasticizer in solid pyrotechnic compositions.

## Patentansprüche

1. Polyglycidylazide mit allgemeiner Formel (I)

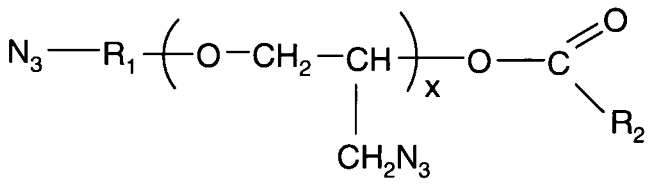

(I)

in welcher:

- $R_1$ eine Alkylenkette darstellt, die 2 bis 4 Kohlenstoffatome umfaßt,
- $R_2$ eine Alkylkette darstellt, die 1 bis 4 Kohlenstoffatome umfaßt,
- x eine ganze Zahl derart darstellt, daß $4 \leq x \leq 10$ ist.

**2.** Polyglycidylazide nach Anspruch 1, **dadurch gekennzeichnet, daß** $R_1$ $-(CH_2)_2-$darstellt und dadurch, daß $R_2$ $-CH_3$ darstellt.

**3.** Polyglycidylazide nach Anspruch 1 oder 2, **dadurch gekennzeichnet, daß** x derart ist, daß $6 \leq x \leq 8$ ist.

**4.** Verfahren zur Synthese von Polyglycidylaziden mit Formel (I)

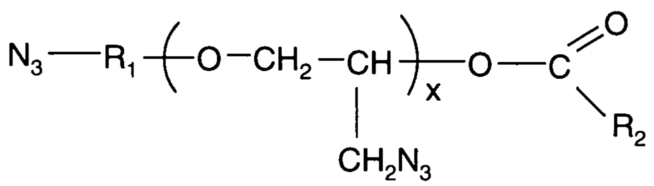

(I)

in welcher:

- $R_1$ eine Alkylenkette darstellt, die 2 bis 4 Kohlenstoffatome umfaßt,
- $R_2$ eine Alkylkette darstellt, die 1 bis 4 Kohlenstoffatome umfaßt,
- x eine ganze Zahl derart darstellt, daß $4 \leq x \leq 10$ ist,

**dadurch gekennzeichnet, daß**

- in einem ersten Schritt ein Polyepichlorhydrin mit Hydroxylterminierung synthetisiert wird, mit allgemeiner Formel (II)

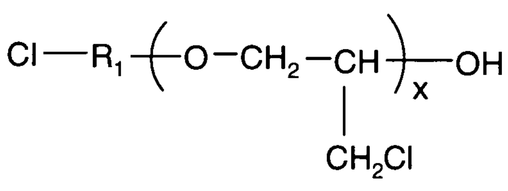

(II)

in welcher x und $R_1$ die oben genannte Bedeutung haben, durch Polymerisation von Epichlorhydrin in Gegenwart eines Alkoholstarters mit allgemeiner Formel (III) $Cl-R_1-OH$, in welcher $R_1$ die oben genannte Bedeutung hat, und dann dadurch, daß

- in einer zweiten Stufe ein Polyepichlorhydrin mit Acyloxyterminierung synthetisiert wird mit allgemeiner Formel (IV)

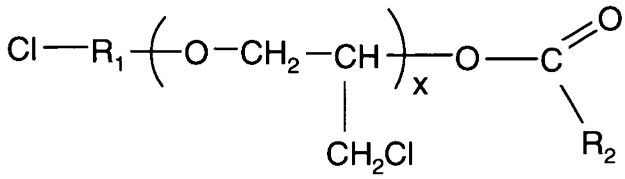

(IV)

in welcher $R_1$, $R_2$ und x die oben genannte Bedeutung haben, durch Reaktion in Gegenwart eines basischen organischen Katalysators, des oben genannten Polyepichlorhydrins mit Hydroxylterminierung mit Formel (II) mit einem Acylierungsreagenz mit allgemeiner Formel (V)

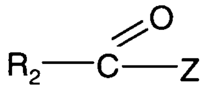

in welcher $R_2$ die oben genannte Bedeutung hat und Z eine Hydroxyl-, Halogenid- oder Acyloxygruppe darstellt, und dadurch, daß

- in einer dritten Stufe eine Azidierung der Chloridfunktionen des oben genannten Polyepichlorhydrins mit Acyloxyterminierung mit allgemeiner Formel (IV), das in der zweiten Stufe erhalten wird, durchgeführt wird.

5. Syntheseverfahren nach Anspruch 4, **dadurch gekennzeichnet, daß** das Acylierungsreagenz mit allgemeiner Formel (V) ein Anhydrid ist mit einer Formel:

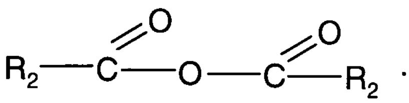

6. Syntheseverfahren nach Anspruch 4 oder 5, **dadurch gekennzeichnet, daß** die drei Reaktionsstufen im organischen Lösungsmittelmedium durchgeführt werden.

7. Syntheseverfahren nach einem der Ansprüche 4 bis 6, **dadurch gekennzeichnet, daß** der basische organische Katalysator gewählt wird aus der Gruppe, die besteht aus den Pyridinen und den Imidazolen.

8. Syntheseverfahren nach einem der Ansprüche 4 bis 7, **dadurch gekennzeichnet, daß** die Azidierung ausgeführt wird mit einem alkalischen Azid.

9. Feste pyrotechnische Zusammensetzung, **dadurch gekennzeichnet, daß** sie ein Polyglycidylazid umfaßt, gemäß einem der Ansprüche 1 bis 3 oder das hergestellt ist gemäß dem Syntheseverfahren von einem der Ansprüche 4 bis 8.

10. Verwendung eines Polyglycidylazids nach einem der Ansprüche 1 bis 3 oder hergestellt nach einem der Ansprüche 4 bis 8 als energiereiches Plastifizierungsmittel in festen pyrotechnischen Zusammensetzungen.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- FR 2707979
- US 4970326 A


### Littérature non-brevet citée dans la description


- *Journal Officiel de la République Française,* 02 Octobre 1980